(19) Europäisches Patentamt
European Patent Office
Office européen des brevets

(11) Numéro de publication : **0 097 667**
**B1**

# (12) FASCICULE DE BREVET EUROPÉEN

(45) Date de publication du fascicule du brevet : **25.03.87**

(51) Int. Cl.[4] : **C 07 H 21/00**, G 01 N 33/50, C 12 N 9/10

(21) Numéro de dépôt : **83900050.2**

(22) Date de dépôt : **29.12.82**

(86) Numéro de dépôt international : **PCT/FR 82/00223**

(87) Numéro de publication internationale : **WO/8302277 (07.07.83 Gazette 83/16)**

(54) **FRAGMENT D'ADN MARQUES A L'UNE AU MOINS DE LEURS EXTREMITES PAR DES RIBONUCLEOTIDES MODIFIES RECONNAISSABLES PAR DES MOLECULES AFFINES ET PROCEDE POUR REALISER UNE ANALYSE DE TELS FRAGMENTS D'ADN.**

(30) Priorité : **29.12.81 FR 8124443**

(43) Date de publication de la demande : **11.01.84 Bulletin 84/02**

(45) Mention de la délivrance du brevet : **25.03.87 Bulletin 87/13**

(84) Etats contractants désignés : **AT BE CH DE GB LI NL**

(56) Documents cités :
**DE-A- 2 618 419**
**DE-A- 2 618 511**
**US-A- 4 255 566**
**Chemical Abstracts, vol. 96, No. 7, February 15, 1982 (Columbus, Ohio, US), P.R. Langer et al.: "Enzymic synthesis of biotin-labeled polynucleotides: novel nucleic acid affinity probes", see page 207, column 2, ref.: 47771z, Proc. Natl. Acad. Sci. U.S.A., 1981, 78(11), 6633-7**

(73) Titulaire : **INSTITUT PASTEUR**
**28, rue du Docteur Roux**
**F-75715 Paris Cedex 15 (FR)**

(72) Inventeur : **KOURILSKY, Philippe**
**207, rue de Vaugirard**
**F-75015 Paris (FR)**
Inventeur : **VINCENT, Christian**
**24, rue du Hameau**
**F-75015 Paris (FR)**
Inventeur : **TCHEN, Paul**
**18, rue du Télégraphe**
**F-92000 Nanterre (FR)**

(74) Mandataire : **Gutmann, Ernest et al**
**S.C. Ernest Gutmann - Yves Plasseraud 67, boulevard Haussmann**
**F-75008 Paris (FR)**

**EP 0 097 667 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention est relative à des fragments d'ADN marqués par l'une au moins de leurs extrémités par des fragments de nucléotides modifiés, plus particulièrement de ribonucléotides modifiés reconnaissables par des molécules affines, et à un procédé pour réaliser une analyse de séquence de tels fragments.

Parmi les techniques d'analyse des séquences de nucléotides contenues dans un ADN, on mentionnera à titre principal la méthode dite de MAXAM et GILBERT (Proc. Natl. Acad. Sci. USA, vol. 74, n° 2, pp. 560-564, février 1977), méthode qui consiste à soumettre l'ADN à analyser, à des réactions de clivages différentiels au niveau des bases guanine et adénine, à des clivages à proportions égales au niveau des bases cytosine et thymine et enfin à des clivages au niveau des seules bases cytosine. Cette méthode implique cependant que l'ADN étudié ait auparavant été marqué à l'une de ses extrémités par un marqueur radioactif, notamment le $^{32}p$, d'où la possibilité, après séparation des fragments obtenus après les susdites opérations de clivage, notamment par électrophorèse sur un gel de polyacrylamide, de reconstituer la séquence d'enchaînement des différents nucléotides dont était initialement constitué l'ADN étudié, plus particulièrement à partir des images autoradiographiques qui peuvent être formées sur un film impressionnable appliqué sur le gel.

Les difficultés qu'implique la révélation autoradiographique des produits des clivages sur la plaque de gel sont bien connues.

L'utilisation de la radioactivité en elle-même n'est pas sans danger. Ceci étant, on avait déjà, notamment pour des raisons semblables, proposé de substituer des marqueurs non radioactifs à un marqueur radioactif dans d'autres procédés qui faisaient couramment usage de ce dernier type de marqueur.

Par exemple, il a été proposé par Pennina R. Langer et al. Proc. Natl. Acad. Sci. USA, vol. 78, n° 11, pp. 6633-6637, novembre 1981, de modifier des sondes d'hybridation de polynucléotides pour la détection de séquences spécifiques d'ADN ou d'ARN. Par translation de coupure (« nick-translation ») en présence de nucléotides modifiées par la biotine et d'une polymérase. L'incorporation aux sondes ainsi modifiées des ribonucléotides modifiés par la biotine n'altère pas leur capacité d'hybridation aux séquences spécifiques d'ADN ou d'ARN qui leur correspondent. La détection de la sonde hybridée peut alors être réalisée par l'intermédiaire de réactifs reconnaissant la biotine, par exemple l'avidine ou des anticorps anti-biotine.

Pour démontrer le niveau d'efficacité de la méthode, les auteurs ont montré qu'un ADN du virus de singe 40 (Simian virus 40) linéarisé par l'enzyme de restriction EcoRI, dont les extrémités adhésives avaient été bicaténarisées en présence de polymérase (fragment de Klenow) et de nucléotides, parmi lesquels des 5-alkylaminobiotinedéoxyuridinetriphosphates, pouvait être sélectivement fixé sur de l'avidine immobilisée sur une agarose réticulée connue sous la désignation SEPHAROSE®.

La demande de brevet allemand DE-A-2 618 511 publiée au nom de Miles Laboratories Inc. décrit un procédé permettant le dosage d'un ligand déterminé dans un milieu, ce procédé mettant en jeu un conjugué formé entre ce ligand et un groupe de marquage possédant une activité dont les variations peuvent être mesurées. L'ATP figure parmi les composés susceptibles de fournir un tel groupe. Le procédé du genre en question comprenant alors la mise en réaction de l'ATP entrant dans la constitution desdits conjugués avec un substrat approprié (par exemple la luciférine réduite), le cas échéant en présence d'une enzyme jouant le rôle de catalyseur (par exemple la luciférase), la mesure de variation de l'activité de l'ATP à l'égard du substrat, et la corrélation de la variation mesurée à l'existence et à la concentration du « ligand » dans le milieu étudié».

Concernant les techniques d'analyse de séquences des nucléotides auxquelles la présente invention se rapporte plus spécialement, il y a lieu de tenir compte d'une difficulté supplémentaire lorsque l'on a recours à des marqueurs radioactifs et notamment au niveau de la révélation autoradiographique des produits de clivage de l'ADN étudié sur la plaque de gel, en raison du rayonnement des isotopes marqués dans toutes les directions de l'espace, une résolution satisfaisante des différentes bandes de migration dans le gel suppose l'utilisation de plaques extrêmement minces. En général l'épaisseur de ces plaques ne dépasse pas 0,3 mm d'épaisseur. L'utilisation de plaques plus épaisses entraînerait une diminution rapide du pouvoir de résolution du procédé autoradiographique. L'utilisation de la radioactivité en elle-même n'est pas non plus sans dangers.

L'invention a pour but de remédier à ces difficultés, notamment de fournir un procédé de modification des ADN dont la séquence doit être étudiée, rendant plus aisée la détection ultérieure des fragments d'ADN susceptibles d'être obtenus par un procédé d'analyse de séquences (tel qu'il a été rappelé plus haut, ou par tout autre procédé susceptible de conduire à des résultats analogues).

L'ADN modifié selon l'invention est caractérisé par un ribonucléotide modifié unique, fixé à l'une au moins de ses extrémités, la modification du ribonucléotide lui-même consistant en une molécule chimique fixée de façon covalente à ce ribonucléotide et portant au moins un groupe non engagé dans la liaison covalente susdite et couplable directement ou indirectement avec une molécule ou un produit ayant une affinité spécifique pour ce groupe et permettant par conséquent la reconnaissance sélective de l'ADN modifié.

En particulier, ce groupe permet une liaison affine chimique ou immunologique avec une molécule

ou un produit lui-même directement révélable ou susceptible d'être révélé à son tour par couplage avec un autre produit révélateur. En outre, ce groupe est tel qu'il ne modifie pas la capacité de ce ribonucléotide à être fixé à l'extrémité d'un ADN, en présence d'une ADN-transférase terminale, lorsqu'il est mis en contact avec cet ADN, dans des conditions permettant la fixation d'un ou de plusieurs ribonucléotides sur cette extrémité.

L'invention concerne également un procédé pour obtenir un tel ADN modifié, ce procédé consistant à traiter l'ADN à étudier avec un tel ribonucléotide modifié, le cas échéant, en présence d'au moins celui des ribonucléotides normalement susceptible de s'apparier dans un hybride ARN : ARN avec le ribonucléotide en question, porteur ou non d'un tel groupe de modification, et d'une ADN-terminale transférase, le cas échéant, et lorsque le produit de fixation à l'extrémité de l'ADN résultant de ce traitement consiste en un oligomère de nucléotides modifiés, à éliminer les ribonucléotides modifiés de cet éventuel oligomère, à l'exception du ribonucléotide directement fixé au déoxyribonucléotide terminal d'extrémité de l'ADN en question. Cette élimination est de préférence faite au moyen d'une base alcaline, notamment de la soude, dans des conditions permettant la séparation des liaisons que forment entre eux les ribonucléotides dans ce fragment d'oligomère.

On peut obtenir de façon en soi connue, à partir d'un ADN portant à ses deux extrémités des ribonucléotides eux-mêmes modifiés, comme défini ci-dessus (ou à partir d'un ADN n'en portant qu'à l'une de ses extrémités), des fragments d'ADN plus petits, soit par action d'agents chimiques susceptibles de provoquer des clivages, notamment au niveau de nucléotides déterminés, soit, de préférence, par action d'endonucléases, notamment d'enzymes de restriction appropriés, dans la mesure où l'ADN correspondant comporte le site de restriction correspondant.

Le traitement de ce fragment d'ADN avec plusieurs enzymes de restriction permet, par récupération des différents fragments marqués à la même extrémité, l'établissement éventuel d'une carte de restriction de cet ADN.

Lorsqu'à une certaine enzyme de restriction correspond un site de restriction unique, le traitement dudit fragment d'ADN par cette enzyme permet l'obtention de fragments de longueur déterminée, se prêtant à une analyse de la séquence des déoxyribonucléotides dont ils sont formés.

En effet, l'invention concerne aussi, dans l'une de ses applications préférées, un procédé comprenant les étapes qui consistent à traiter un ADN ainsi marqué à l'une de ses extrémités par un ribonucléotide modifié comme ci-dessus défini, avec des agents chimiques permettant d'opérer au sein de cet ADN des clivages différentiels au niveau de certaines bases, notamment celles qui ont été décrites par Maxam & Gilbert dans l'article déjà mentionné plus haut, à recueillir et à séparer des fragments d'ADN dans un système permettant de les trier par ordre de tailles, et à les faire réagir avec des réactifs permettant la réalisation d'un couplage des groupes chimiques portés par des ribonucléotides terminaux de ceux des fragments qui en portent, avec une molécule ou un produit ayant une affinité sélective à l'égard du groupe chimique de modification desdits ribonucléotides terminaux.

Les ribonucléotides modifiés fixés à l'extrémité des ADN concernés sont principalement dérivés :

— de l'acide adénosine 5'-triphosphorique (ATP),
— de l'acide guanosine 5'-triphosphorique (GTP),
— de l'acide cytidine 5'-triphosphorique (CTP) et
— de l'acide uridine 5'-triphosphorique (UTP).

Le groupe chimique lié de façon covalente à ces ribonucléotides peut revêtir les formes les plus diverses, dès lors qu'il possède un groupe permettant son couplage avec des substances affines permettant sa révélation, de préférence sous forme directement visualisable, et qu'il n'empêche pas une ADN-transférase terminale d'assurer la fixation des ribonucléotides modifiés obtenus aux extrémités d'un ADN.

Parmi les groupes chimiques préférés susceptibles d'être fixés sur les bases des susdits ribonucléotides, on mentionnera tout groupe susceptible d'être reconnu spécifiquement par une autre molécule ou par un produit, lui-même aisément détectable, de préférence par une méthode de visualisation.

Cette autre molécule ou cet autre produit consiste par exemple en une enzyme, dont la présence peut être révélée par l'action qu'elle est susceptible d'exercer sur un substrat, de préférence un substrat donnant lieu à des réactions de coloration ou de décoloration, ou plus généralement encore de modification de spectres d'absorption respectivement décelables par colorimétrie ou spectrophotométrie. On peut naturellement avoir recours à des molécules ou produits donnant lieu à des réactions de fluorescence, à des modifications de densité optique, par exemple des produits comprenant des groupes dérivés de l'aminofluorène, du chlorure de dansyle ou de la rhodamine.

Parmi les groupes de modification appropriés, on mentionnera les groupes chimiques dont est connue l'affinité pour un autre type de molécule chimique. Parmi ces groupes de modification chimique, on peut mentionner la biotine ou l'avidine, dont on connaît les affinités réciproques, les groupes dérivés de l'une de ces molécules pouvant servir à la modification du ribonucléotide choisi et l'autre de molécule coupable avec un réactif marqué par une enzyme ou susceptible d'être fixée à une enzyme, par exemple dans les conditions décrites dans le brevet FR-A-2422956 de l'INSTITUT PASTEUR déposé le 13 avril 1978. Ce réactif consiste par exemple en un anticorps spécifique dirigé contre le groupe de modification ou en une molécule ayant une affinité spécifique pour ledit groupe de modification.

Parmi les groupes de modification utiles du ribonucléotide initial, figurent également des antigènes

ou haptènes susceptibles d'être reconnus par des anticorps préalablement formés contre ces antigènes ou contre ces haptènes, plus particulièrement lorsque ceux-ci ont été fixés au préalable sur une macromolécule support, telle qu'une sérum-albumine ou un polypeptide, par exemple une polylysine. Parmi ces antigènes ou haptènes, on peut citer la biotine et l'avidine elles-mêmes, des groupes acétylaminofluorène, des peptides, hormones ou prostaglandines, notamment ceux auxquels correspondent des antisérums ou anticorps spécifiques, des lectines, dont est connue la capacité à être couplées à des enzymes permettant leur révélation, notamment des péroxydases ou des β-galactosidases. De tels sérums ou anticorps sont disponibles dans le commerce.

Mais la molécule ou le produit présentant des caractéristiques d'affinité vis-à-vis du groupe de modification susdit, sert éventuellement aussi seulement de relais vis-à-vis d'une autre molécule ou d'un autre produit susceptible d'être visualisé à son tour, notamment dans les conditions sus-indiquées ; par exemple le produit présentant les caractéristiques d'affinité, vis-à-vis du groupe de modification susdit, est constitué par un anticorps lui-même non marqué, mais reconnaissable à son tour par des anticorps contre lui-même, ces derniers anticorps étant eux-mêmes couplés à l'enzyme susceptible d'agir sur un substrat spécifique, dans les conditions classiques en matière de dosage immunoenzymatique.

D'une façon générale, le groupe de modification du ribonucléotide est constitué par toute molécule ou produit chimique fixable sur un ribonucléotide et ensuite détectable dans les conditions qui ont été indiquées, dans la mesure où il ne perturbe pas la capacité du nucléotide modifié obtenu à être attaché à l'extrémité d'un ADN, sous l'action d'une ADN-transférase terminale ; cette propriété est également à la base du test de reconnaissance qui permet d'en constater la réalité, savoir la visualisation effective, notamment dans les conditions sus-indiquées, d'un fragment d'ADN déterminé porteur à l'une au moins de ses extrémités du ribonucléotide modifié par la molécule ou produit étudié, dans la mesure où un tel ADN transformé aura été formé, à l'issue de la réaction du fragment d'ADN correspondant avec le ribonucléotide modifié, en présence d'une ADN-transférase terminale dans des conditions permettant normalement la fixation du même ribonucléotide non modifié ou d'un oligomère de tels ribonucléotides, à l'une au moins des extrémités de ce fragment d'ADN.

Lorsque le ribonucléotide initial est constitué par l'ATP, le groupe de modification chimique sus-indiqué est fixé sur la position 6, de préférence 8, du groupe adénine.

Cette fixation intervient avantageusement par l'intermédiaire d'un bras du type $—NH—(CH_2)_x—X$, ou $—CO—(CH_2)_x—X$, dans lequel x varie de 2 à 20, notamment de 6 à 12, et X est un groupe assurant la liaison entre un groupe M, choisi parmi ceux qui sont susceptibles d'une réaction de liaison avec un agent chimique ou immunologique ayant une affinité sélective pour ce groupe. Les groupes $CH_2$ du bras susdit peuvent en partie être remplacés par des groupes CO ou NH, à condition naturellement que ces groupes de remplacement ne soient pas adjacents à des groupes identiques.

Par exemple, si l'on considère le cas de la modification du groupe adénine de l'ATP en sa position 8, le ribonucléotide modifié obtenu peut être représenté par la formule (cas d'un bras du type $—NH—(CH_2)_x—X—$) :

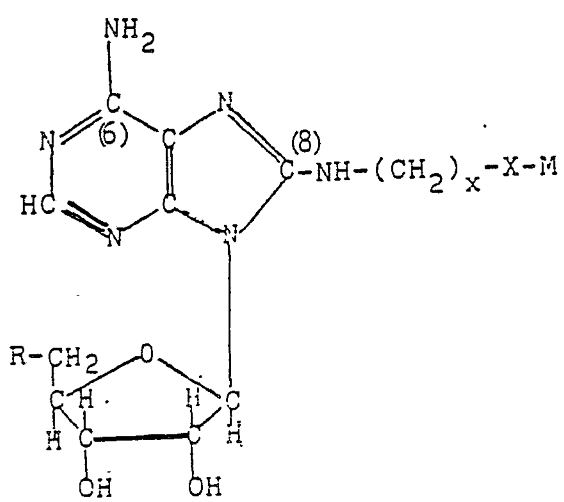

(I)

dans laquelle R est un groupe triphosphate, x, X et M ont les significations sus-indiquées. Avantageusement, le groupe X est constitué par un groupe NH ou CO.

Un procédé pour fabriquer le dérivé de formule I, par exemple à partir de l'ATP préalablement bromé en position 8, consiste à le faire réagir dans les conditions appropriées, avec un composé de formule $H_2N—(CH_2)_x—X—Y$, dans lequel Y représente un radical auquel sera ensuite substitué le groupe M susdit, notamment par la mise en œuvre d'une réaction de condensation avec une molécule MZ, au cours de laquelle est alors formé le produit de condensation de formule I avec libération d'une molécule Y—Z.

Lorsque le groupe X est NH, Y est avantageusement de l'hydrogène. Lorsque X est CO, Y est avantageusement un hydroxyle. Z peut être constitué par tout autre groupe susceptible d'être détaché de

M dans la susdite réaction de condensation, par exemple le fluor lorsque la molécule chimique donneuse du groupe recherché est le fluoro-1-dinitro-2,4 benzène, ou un hydroxyle ou de l'hydrogène dans le cas d'un peptide. Dans ce dernier cas, la fixation de ce peptide à l'extrémité du bras susdit peut, lorsque le groupe XY est constitué par un groupe $NH_2$ ou COOH, être effectué par la mise en œuvre de réactions de couplage, traditionnelles dans la chimie des protéines, entre groupes carboxyle et amine, respectivement portés par les deux éléments peptidiques distincts à coupler, par exemple par condensation en présence d'un agent de condensation, tel que le dicyclohexyl-carbodiimide ou après formation préalable d'un ester activé à la fonction carboxylique de celui de ces deux éléments peptidiques qui le portent.

La position 8 sur le cycle adénine de l'ATP ne constitue évidemment pas le seul point sur lequel peut se brancher une chaîne porteuse d'un groupe de modification tel qu'il a été défini plus haut. A titre d'exemple, on peut également substituer l'un des atomes d'hydrogène porté par le carbone en position 6 du cycle adénine par une chaîne porteuse de ce groupe. A titre d'exemple, on mentionnera la possibilité de substitution qui consiste à faire réagir avec l'ATP de l'acide iodoacétique ou un acide organique iodé équivalent, permettant la formation préalable d'un sel quaternaire, faisant intervenir l'azote en position 1, sel qui se transforme ensuite par chauffage en milieu basique à 35 °C, à pH légèrement basique, notamment à pH 8, pendant le temps suffisant, par exemple 72 heures, en un produit de substitution de l'un des hydrogènes du groupe $NH_2$ fixé sur le carbone en position 6 du groupe adénine (réaction du type connu sous l'expression « réarrangement de Dimroth »).

On obtient alors (lorsque l'acide organique iodé est constitué par l'acide iodoacétique) le composé de formule II ci-après :

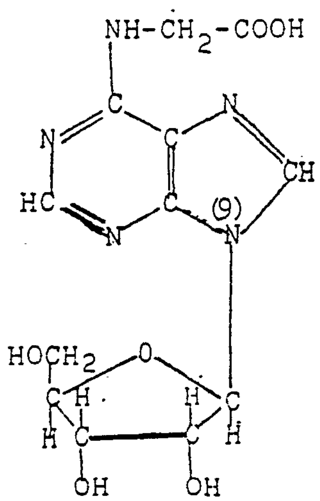

(II)

Ce composé peut ensuite être transformé, par réaction avec le composé de formule $H_2N—(CH_2)_x—X—Y$ déjà défini plus haut, dans des conditions permettant la liaison chimique entre le groupe carbonyle initialement contenu dans le groupe carboxyle dans le composé de formule II et le groupe imino appartenant initialement à la fonction aminée du composé $H_2N—(CH_2)_x—X—Y$, lequel peut alors être couplé à son tour avec un composé de formule MZ, dans les conditions qui ont déjà été définies plus haut.

Il va de soi que tous les éléments qui précèdent ne visent qu'à illustrer des modes de préparation particuliers qui permettent de fixer sur l'ATP un groupe de modification choisi parmi ceux auxquels correspondent des molécules affines, comme cela a été défini plus haut.

On peut de façon analogue fabriquer des dérivés du GTP, les groupes de modification chimique sus-définis étant alors fixés dans des conditions analogues sur la position 2 ou de préférence 8 du groupe guanine du GTP. Les mêmes mécanismes de réaction sont normalement applicables.

De même, on peut mettre en œuvre, dans les applications préférées qui ont été indiquées, de l'UTP ou du CTP modifié par un groupe chimique répondant aux conditions sus-indiquées, en ayant cependant recours au procédé tout à fait différent décrit dans l'article de P. R. LANGER et al (Proc. Natl. Acad. Sci. USA, vol. 78, N° 11, p. 6633-6637, novembre 1981).

D'autres caractéristiques de l'invention apparaîtront encore au cours de la description d'exemples de mises en œuvre préférées de l'invention.

Préparation du 8-{N-[6-(2',4'-dinitrophenylamino)hexyl]amino}adénosine 5'-triphosphate.

On fait réagir du 8-[(ω-aminohexyl)amino]adénosine 5'-triphosphate avec du fluoro-1-dinitro-2,4-benzène, au sein d'un mélange eau-éthanol 10/1 volumes %, à pH 8,8, à 40 °C, et en présence d'un sel, notamment chlorure de magnésium. Le produit de réaction finalement obtenu a pour formule :

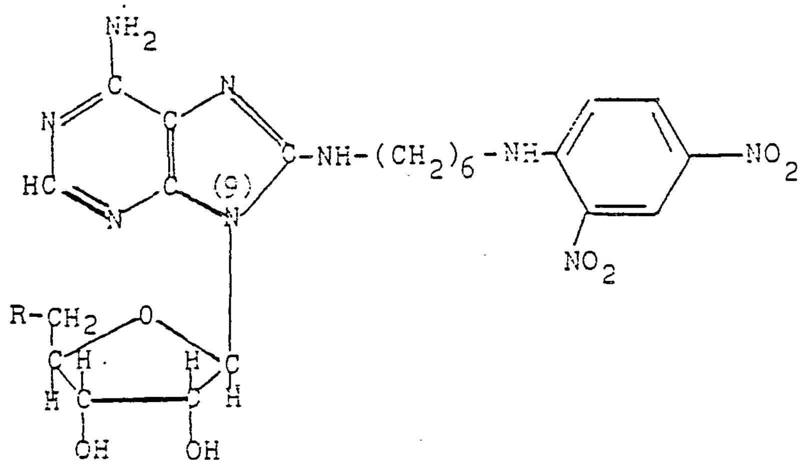

(III)

On récupère le dérivé de formule III, ci-après dénommé ATP-DNP, après une purification comprenant la fixation du ribonucléotide sur DEAE cellulose, élution avec un gradient de LiCl 0,2 N, pH 5,5, à LiCl 0,5 N, pH 2 et filtration sur tamis moléculaire du type SEPHADEX® G50. Le rendement réactionnel est de 52 %. Les fractions recueillies sont analysées par spectrophotométrie d'absorption de rayonnements de longueurs d'onde de 280 et 360 nanomètres respectivement. On recueille celle des fractions dont les densités optiques dans les deux susdits domaines de longueurs d'onde sont dans un rapport $(DO_{280}/DO_{360})$ égal à 4. Le produit contenu dans cette fraction correspond à celui résultant de la fixation de 1 mole de DNP sur 1 mole d'ATP. Il ne donne également qu'une tache dans un système de chromatographie en couche mince. Le produit de cette fraction est lyophilisé.

Ce produit présente la caractéristique d'être reconnu par des anticorps formés au préalable à l'égard du dinitro-2,4 benzène, qui avait préalablement été fixé sur un support macromoléculaire du type de la sérum-albumine. Des anticorps de ce type sont par ailleurs disponibles dans le commerce.

Préparation du 8-[6-(N-biotinyl-amino)hexylamino]adénosine 5'-triphosphate.

On condense du 8-[(ω-amino-hexyl)amino]adénosine-5'-triphosphate avec le biotinyl-N-hydroxysuccinimideester, dans les conditions décrites par LANGER et al, telles qu'appliquées à la fabrication du biotinyl-UTP à partir de la 5-[(3-amino)allyl]uridine. On obtient alors le composé de formule :

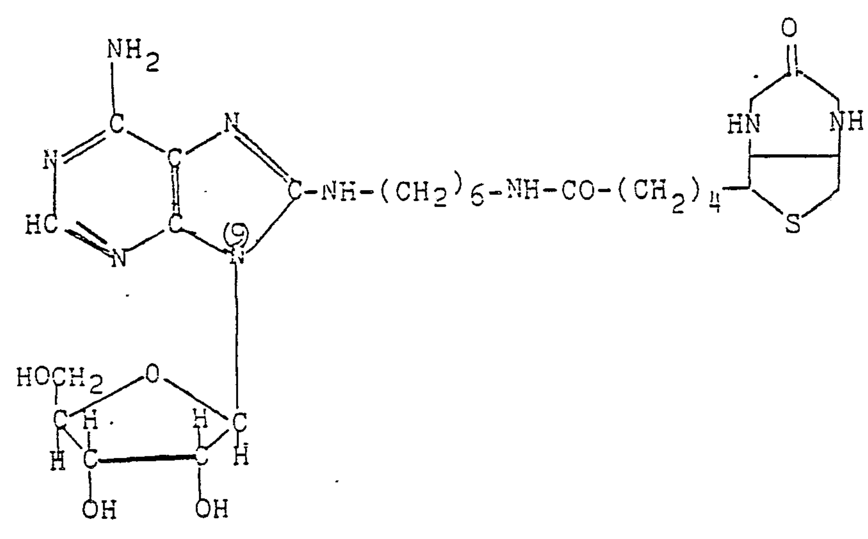

(IV)

Fabrication d'un ADN marqué à ses extrémités par des ribonucléotides modifiés.

600 micromoles d'ATP-DNP et 10 microgrammes d'un fragment d'ADN comportant 500 paires de bases sont mis à réagir en présence de 30 unités d'ADN-terminal-transférase à 37 °C et pendant 24 heures, au sein d'une solution tampon, dont la composition est la suivante (volume final 200 microlitres) :

| | |
|---|---|
| cacodylate de potassium : | 100 mM |
| sérum d'albumine bovine : | 1 mg/ml |
| dithiothreitol : | 1 mM |
| chlorure de cobalt : | 1 mM |

L'ADN retenant à ses extrémités des groupes ATP-DNP est purifié, par passage de la solution sur une colonne du tamis moléculaire commercialisé sous la marque SEPHADEX® G 50.

Une goutte de cette fraction est déposée sur un filtre de cellulose. Après séchage du filtre, on met celui-ci au contact d'une solution d'anticorps anti-DNP de lapin. L'anticorps non fixé est rincé. Le filtre est ensuite mis au contact d'une solution d'anticorps de lapin liée à de la péroxydase. Après rinçage de l'excès d'anticorps non fixé, on révèle la présence d'anticorps fixés sur le filtre avec une solution d'un substrat pour la péroxydase. Cette solution contient :

— de l'eau oxygénée : 10 µl d'$H_2O_2$ à 110 volumes,
— de l'acétate de potassium : 9,5 ml 0,05 M, pH 5,1,
— du carbazole : 2 mg pour 0,5 ml de N-N'-diméthylformamide.

La présence de l'anti-anticorps de lapin sur le filtrat est alors révélé par la formation d'un précipité brun rouge.

La sensibilité de la méthode est telle qu'elle permet de détecter des quantités extrêmement faibles d'ADN, notamment $380 \cdot 10^{-4}$ picomoles.

Application des ADN modifiés conformes à l'invention à l'analyse des séquences de déoxynucléotides qui les constituent.

Les premières étapes de la méthode permettant l'analyse des séquences de nucléotides contenus à l'intérieur d'un ADN déterminé à double-brin sont schématisées ci-après.

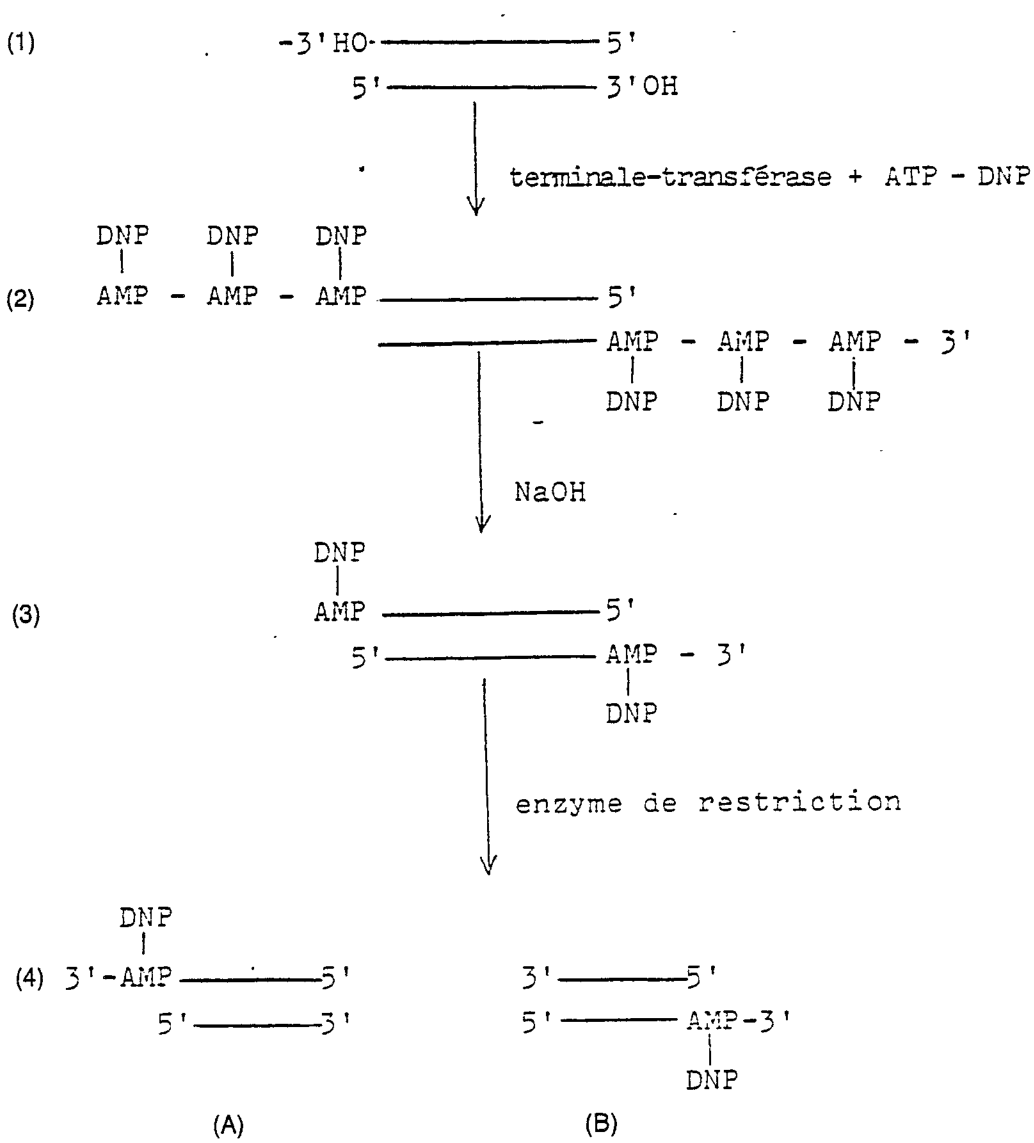


L'ADN initial (1) est schématisé par deux traits parallèles comportant l'indication des extrémités respectives 3', 5' de chacun des brins de cet ADN.

Par réaction avec l'ATP-DNP, en présence d'ADN-terminale-transférase, on obtient le fragment d'ADN

modifié représenté en (2), dont les extrémités 3′ portent des oligomères d'AMP-DNP (trois motifs AMP-DNP par oligomère, dans le cas de l'exemple considéré ; avec AMP désignant les éléments dérivés du ATP-DMP après l'élimination de deux groupes phosphate par monomère d'ATP-DMP, du fait de l'oligomérisation).

L'ADN ainsi modifié est soumis à une hydrolyse alcaline au sein d'une solution de soude 1 M, à 40 °C, ce grâce à quoi on obtient un ADN modifié, à nouveau récupérable par filtration à travers un tamis moléculaire, ne portant plus à chacune de ses extrémités terminales qu'un seul ribonucléotide modifié (3).

Par action d'une enzyme de restriction, par exemple dans les conditions décrites par MAXAM & GILBERT, on produit deux fragments repérés par A et B en (4). On remarquera que l'on peut évidemment modifier à volonté l'ordre des opérations (2), (3) et (4).

Après isolement et purification par exemple des fragments B, ceux-ci sont répartis en plusieurs lots respectivement soumis aux différentes réactions de clivage différentiel, telles que celles et dans les conditions décrites par MAXAM & GILBERT. Les produits résultant de ces réactions de clivage sélectif sont soumis à des opérations d'électrophorèse sur gel d'acrylamide, dans les conditions décrites par les mêmes auteurs, pour séparer les différents fragments d'ADN, respectivement marqués à leurs extrémités par ordre de tailles, et en différentes bandes.

Conformément à l'invention, on procède ensuite à la révélation des différents fragments marqués par exemple *in situ* dans le gel, par mise en contact de ces derniers avec les solutions d'anticorps, dans les conditions qui ont été indiquées plus haut. On peut aussi transférer au moins en partie les bandes de migrations distinctes sur un filtre de cellulose ou support analogue, par application de ce filtre sur le gel, les différents fragments ou bandes étant alors détectés sur le filtre lui-même, par exemple dans les conditions qui ont déjà été indiquées plus haut.

On appréciera que cette méthode extrêmement sensible n'implique plus l'utilisation antérieurement nécessaire de plaques de gel extrêmement minces, puisque la détection peut maintenant s'effectuer par visualisation directe des bandes de fractionnement soit dans le gel, soit sur le filtre.

**Revendications**

1. ADN modifié à au moins l'une de ses extrémités par un ribonucléotide lui-même modifié par un groupe de modification consistant en une molécule chimique lié de façon covalente à ce ribonucléotide et portant au moins un groupe non engagé dans la liaison covalente susdite et couplable directement ou indirectement avec une molécule ou un produit ayant une affinité spécifique pour ce groupe et permettant sa reconnaissance, ledit groupe étant en outre tel qu'il n'empêche pas le ribonucléotide qui le porte à être fixé à l'extrémité d'un ADN, en présence d'une ADN-transférase terminale, lorsqu'il est mis en contact avec cet ADN, dans des conditions permettant la fixation d'un ou de plusieurs ribonucléotides sur cette extrémité.

2. ADN modifié selon la revendication 1, caractérisé en ce que le susdit groupe de modification est susceptible d'être reconnu spécifiquement et directement par une autre molécule ou par un produit, lui-même aisément détectable, par une méthode de visualisation.

3. ADN modifié selon la revendication 2, caractérisé en ce que le susdit groupe de modification est constitué par un antigène ou un haptène susceptible d'être reconnu par un anticorps préalablement formé contre cet antigène ou contre cet haptène.

4. ADN modifié selon la revendication 1, caractérisé en ce que le susdit groupe de modification sert de relais vis-à-vis d'une autre molécule ou d'un autre produit susceptible d'être visualisé à son tour.

5. ADN modifié selon l'une quelconque des revendications 1 à 4, caractérisé en ce que le susdit groupe de modification ou, selon le cas, la susdite molécule relais, est coupable chimiquement avec une molécule affine marquée par une enzyme ou immunologiquement avec un anticorps marqué par une enzyme et ayant une affinité sélective pour ledit groupe de modification ou la susdite molécule relais.

6. ADN modifié selon l'une quelconque des revendications 1 à 5, caractérisé par la fixation à l'une au moins de ses extrémités d'un groupe de modification fixé de façon covalente sur la position 6, ou de préférence 8, de son groupe adénine, par l'intermédiaire d'un bras du type $—NH—(CH_2)_x—X$, ou $—CO(CH_2)_x—X$, dans lequel x varie de 2 à 20, notamment de 6 à 12, et X est un groupe assurant la liaison entre un groupe M choisi parmi ceux qui sont susceptibles d'une réaction de liaison avec un agent chimique ou immunologique ayant une affinité sélective pour ce groupe.

7. ADN modifié selon la revendication 6, caractérisé en ce que les groupes $CH_2$ du bras susdit peuvent en partie être remplacés par des groupes CO ou NH, à condition naturellement que ces groupes de remplacement ne soient pas adjacents à des groupes identiques.

8. ADN modifié selon l'une quelconque des revendications 1 à 7, caractérisé en ce que le susdit groupe de modification comprend un groupe dérivé de la biotine, de l'avidine ou un groupe dinitro-2,4-phényle.

9. Procédé de modification d'un ADN comprenant les étapes consistant à traiter ledit ADN avec un ribonucléotide, portant un groupe de modification tel que défini dans l'une quelconque des revendications 1 à 8, en présence d'une ADN-terminaltransférase, puis le cas échéant, et lorsque le produit de

fixation à l'extrémité de l'ADN résultant de ce traitement consiste en un oligomère de nucléotides modifiés, à éliminer les ribonucléotides modifiés de cet éventuel oligomère, à l'exception du ribonucléotide directement fixé au déoxyribonucléotide terminal d'extrémité de l'ADN en question, de préférence par l'action d'une base alcaline, notamment de l'hydroxyde de sodium, dans des conditions permettant la séparation des liaisons que forment entre eux les ribonucléotides dans ce fragment d'oligomère.

10. Application de la modification de l'ADN à l'une de ses extrémités par un ribonucléotide portant un groupe de modification tel que défini dans l'une quelconque des revendications 1 à 8, à l'obtention d'une carte de restriction de cet ADN par action des enzymes de restriction correspondantes, et par récupération, tri et comparaison des tailles des fragments obtenus et portant tous le même ribonucléotide modifié à l'une de leurs extrémités.

11. Application de la modification de l'ADN à l'une de ses extrémités par un ribonucléotide portant un groupe de modification tel que défini dans l'une quelconque des revendications 1 à 8, à la réalisation d'une analyse de la séquence de nucléotides dont est constitué cet ADN, par un procédé comprenant les étapes consistant à traiter cet ADN avec des agents chimiques permettant d'opérer au sein de cet ADN des clivages différentiels au niveau de certaines bases, à recueillir et à séparer des fragments d'ADN dans un système permettant de les trier par ordre de tailles, et à les faire réagir avec des réactifs permettant la réalisation d'un couplage des groupes chimiques portés par les ribonucléotides terminaux de ceux des fragments qui en portent, avec une molécule ou un produit ayant une affinité sélective à l'égard du groupe chimique de modification desdits ribonucléotides terminaux, en vue de leur visualisation, et à déterminer les nucléotides terminaux non modifiés de chacun de ces fragments d'ADN, eu égard à la nature de l'agent chimique utilisé pour le clivage à son niveau.

**Claims**

1. DNA modified at at least one end by a ribonucleotide which is itself modifed by a modification group consisting in a chemical molecule forming a covalent bond with said ribonucleotide and bearing at least one group not involved in the said covalent bond and couplable directly or indirectly with a molecule or a product having a specific affinity for this group and allowing recognition of it, the said group being furthermore such that it does not prevent, the ribonucleotide bearing it from being fixed at the end of a DNA, in the presence of a terminal transfer-DNA, when it comes into contact with that DNA, under conditions which permit the fixation of one or more ribonucleotides to that end.

2. DNA modified according to claim 1, characterised in that the above-mentioned modification group is capable of being recognised specifically and directly by another molecule or by a product, easily detectable in itself, by a visualisation method.

3. DNA modified according to claim 2, characterised in that the above-mentioned modification group consists of an antigen or a hapten capable of being recognised by a previously formed antibody against said antigen or hapten.

4. DNA modified according to claim 1, characterised in that the above-mentioned modification group acts as a relay in relation to another molecule or another product capable of being visualised in its turn.

5. DNA modified according to any one of the claims 1 to 4, characterised in that the above-mentioned modification group or, in the appropriate case, the above-mentioned relay molecule is chemically couplable with an affine molecule marked by an enzyme or immunologically with an antibody marked by an enzyme and having a selective affinity for the said modification group or the above-mentioned relay molecule.

6. DNA modified according to any one of the claims 1 to 5, characterised by the fixation at at least one end of a modification group fixed covalently to position 6, or preferably 8, of its adenine group, by means of an arm of the type $-NH-(CH_2)_x-X$ or $-CO(CH_2)_x-X$, where x varies between 2 and 20, and especially between 6 and 12, and X is a group ensuring the bond between an M group chosen from amongst those which are capable of a bonding reaction with a chemical agent or immunological agent having a selective affinity for said group.

7. DNA modified according to claim 6, characterised in that the $CH_2$ groups of the above-mentioned arm can in part be replaced by CO or NH groups, on condition, naturally, that these replacement groups are not adjacent to identical groups.

8. DNA modified according to any of the claims 1 to 7, characterised in that the above-mentioned modification group contains a group derived from biotin, avidin or a dinitro-2,4-phenyl group.

9. Process for modifying a DNA consisting of stages wherein the said DNA is treated with a ribonucleotide bearing a modification group such as described in claims 1 to 8, in the presence of a terminal transfer-DNA, and wherein subsequently, if necessary, and when the fixation product at the end of the DNA resulting from said treatment consists of an oligomer of modified nucleotides, the modified ribonucleotides of this oligomer are eliminated, with the exception of the ribonucleotide fixed directly to the end terminal deoxyribonucleotide of the DNA in question, preferably through the action of an alkaline base, in particular sodium hydroxide, under conditions allowing the separation of the bonds which the ribonucleotides form between themselves in this oligomer fragment.

10. Application of the modification of the DNA at one end by a ribonucleotide bearing a modification

group such as defined in any one of the claims 1 to 8, to obtaining a restriction chart for this DNA through the action of enzymes of corresponding restriction and by recuperation, sorting and comparison of the sizes of fragments obtained and all of which bear the same modified ribonucleotide at one end.

11. Application of the modification of the DNA at one end by a ribonucleotide bearing a modification group such as defined in any one of the claims 1 to 8, to carrying out an analysis of the sequence of nucleotides of which this DNA is composed, by a process consisting of stages wherein this DNA is treated with chemical agents making it possible to perform within the DNA differential cleavages at the level of certain bases, the fragments of DNA are collected up and separated in a system making it possible to sort them in order of size, and they are reacted with reagents which make it possible to couple the chemical groups borne by the terminal ribonucleotides of those of the fragments which bear them with a molecule or a product having a selective affinity for the chemical modification group of the said terminal ribonucleotides, with a view to visualising them, and the non-modified terminal nucleotides of each of these DNA fragments are determined, on the basis of the nature of the chemical agent used for the cleavage at its level.

**Patentansprüche**

1. DNA, dadurch gekennzeichnet, daß sie an mindestens einem ihrer Enden durch ein Ribonucleotid modifiziert ist, das selbst durch eine aus einem chemischen Molekül bestehende Modifizierungsgruppe modifiziert ist, die kovalent mit dem Ribonucleotid verbunden ist und mindestens eine nicht an der kovalenten Bindung beteiligte Gruppe trägt, die entweder direkt oder indirekt mit einem Molekül oder Produkt koppelbar ist, das eine für diese Gruppe spezifische Affinität aufweist und ihre Wiedererkennung gestattet, wobei die Gruppe auch nicht verhindert, daß das sie tragende Ribonucleotid, wenn es mit einer DNA in Berührung kommt, an ein Ende dieser DNA in Gegenwart einer terminalen DNA-Transferase unter Bedingungen gebunden wird, die die Bindung eines oder mehrerer Ribonucleotide an dieses Ende gestatten.

2. Modifizierte DNA nach Anspruch 1, dadurch gekennzeichnet, daß die Modifizierungsgruppe in einem Verfahren zur Sichtbarmachung durch ein anderes Molekül oder Produkt, das selbst leicht nachweisbar ist, spezifisch und direkt wiedererkennbar ist.

3. Modifizierte DNA nach Anspruch 2, dadurch gekennzeichnet, daß die Modifizierungsgruppe aus einem Antigen oder Hapten gebildet wird, das von einem zuvor gegen dieses Antigen oder Hapten gebildeten Antikörper wiedererkennbar ist.

4. Modifizierte DNA nach Anspruch 1, dadurch gekennzeichnet, daß die Modifizierungsgruppe als Abgrenzung gegenüber einem anderen Molekül oder Produkt dient, das seinerseits wiedererkennbar ist.

5. Modifizierte DNA nach einem der Ansprüche 1 bis 4, dadurch gekennzeichnet, daß die Modifizierungsgruppe oder gegebenenfalls das der Abgrenzung dienende Molekül mit einem Molekül chemisch verbindbar ist, das eine Affinitätsmarkierung mit einem Enzym aufweist, oder das immunologisch mit einem durch ein Enzym markierten Antikörper markiert ist, und das eine selektive Affinität für die Modifizierungsgruppe oder das Abgrenzungsmolekül aufweist.

6. Modifizierte DNA nach einem der Ansprüche 1 bis 5, dadurch gekennzeichnet, daß an mindestens einem ihrer Enden eine Modifizierungsgruppe kovalent an Position 6, vorzugsweise an Position 8, ihrer Adeningruppe gebunden ist durch Zwischenschaltung eines Arms mit der Formel $—NH—(CH_2)_x—X$ oder $—CO(CH_2)_x—X$, in der x den Wert 2 bis 20 hat, vorzugsweise 6 bis 12, und X eine Gruppe ist, die die Verbindung mit einer Gruppe M gewährleistet, die chemisch oder immunologisch mit einem Stoff eine Verbindungsreaktion eingehen kann, der eine für diese Gruppe selektive Affinität aufweist.

7. Modifizierte DNA nach Anspruch 6, dadurch gekennzeichnet, daß die $CH_2$-Gruppen des Arms teilweise durch CO- oder NH-Gruppen mit der Maßgabe ersetzbar sind, daß die Ersatzgruppen nicht neben identischen Gruppen liegen.

8. Modifizierte DNA nach einem der Ansprüche 1 bis 7, dadurch gekennzeichnet, daß die Modifizierungsgruppe eine von Biotin oder Avidin abgeleitete Gruppe oder eine 2,4-Dinitrophenyl-Gruppe enthält.

9. Verfahren zur Modifikation einer DNA, bei dem man die DNA in Gegenwart einer terminalen DNA-Transferase mit einem Ribonucleotid behandelt, das eine Modifizierungsgruppe nach einem der Ansprüche 1 bis 8 trägt und, sobald das dabei entstehende und an das Ende der DNA gebundene Produkt aus einem Oligomer aus modifizierten Nucleotiden besteht, diese modifizierten Nucleotide des Oligomers mit Ausnahme des am terminalen Desoxyribonucleotid des betreffenden DNA-Endes direkt fixierten Ribonucleotid, vorzugsweise durch eine alkalische Base, besonders bevorzugt durch Natriumhydroxyd, bei Bedingungen abspaltet, die die Spaltung der zwischen den Ribonucleotiden und dem Oligomerfragment entstandenen Verbindung gestatten.

10. Verwendung der Modifikation durch ein Ribonucleotid das eine Modifizierungsgruppe nach einem der Ansprüche 1 bis 8 trägt an einem der DNA-Enden zur Erstellung einer Restriktionskarte der DNA mit entsprechenden Restriktionsenzymen durch Wiedergewinnung, Sortieren und Vergleichen der Größe der erhaltenen Fragmente, die noch ein modifiziertes Ribonucleotid an einem ihrer Enden tragen.

11. Verwendung der Modifikation durch ein Ribonucleotid das eine Modifizierungsgruppe nach

einem der Ansprüche 1 bis 8 trägt an einem der DNA-Enden zur Durchführung einer Analyse der Nucleotidsequenz aus der die DNA besteht, durch ein Verfahren, bei dem man DNA mit chemischen Mitteln behandelt, die die differenzierte Abspaltung an bestimmten Basen innerhalb der DNA gestatten ; die DNA-Fragmente in einem System sammelt und trennt, das ein größenabhängiges Sortieren gestattet, und die Fragmente mit Reagenzien umsetzt, die die Entstehung einer Bindung zwischen den von den End-Ribonucleotiden der Fragmente getragenen chemischen Gruppen und einem Molekül oder Produkt erlauben, das in Bezug auf ihre Sichtbarkeit eine für die chemische Modifizierungsgruppe der End-Ribonucleotide selektive Affinität aufweist, und die nicht-modifizierten End-Nucleotide aller DNA-Fragmente in Abhängigkeit der Art des zu seiner Spaltung verwendeten chemischen Mittels bestimmt.